Europäisches Patentamt

European Patent Office

Office européen des brevets

(19)

(11) Veröffentlichungsnummer : **0 079 009**
**B1**

(12) **EUROPÄISCHE PATENTSCHRIFT**

(45) Veröffentlichungstag der Patentschrift :
17.07.85

(21) Anmeldenummer : 82110012.0

(22) Anmeldetag : 29.10.82

(51) Int. Cl.⁴ : **A 61 B   5/04**, H 03 H  11/12,
H 03 F   1/42

(54) EKG-Verstärkerschaltung.

(30) Priorität : 05.11.81 DE 3143831

(43) Veröffentlichungstag der Anmeldung :
18.05.83 Patentblatt 83/20

(45) Bekanntmachung des Hinweises auf die Patenterteilung : 17.07.85 Patentblatt 85/29

(84) Benannte Vertragsstaaten :
BE CH DE FR GB IT LI NL

(56) Entgegenhaltungen :
DE-A- 2 245 224
US-A- 3 611 174
MEDICAL AND BIOLOGICAL ENGINEERING AND
COMPUTING, Band 15, Nr. 4, Juli 1977, Seiten 450-
454, Stevenage, G.B. M.V. THOMAS: "Microelectrode
amplifier with improved method of input-capacitance
neutralisation"
1980 IEEE INTERNATIONAL SYMPOSIUM ON CIR-
CUITS AND SYSTEMS PROCEEDINGS, Band 2, 28.-
30. April 1980, Shamrock-Hilton Hotel, Houston,
Texas R.L. GEIGER: "Design of active filters independent of first- and second-order operational amplifier
time constants effects", Seiten 549-552
NEUES AUS DER TECHNIK, Nr. 1/2, 1. März 1969,
Seite 3, Vogel Verlag, Würzburg, DE. "Biologischer
Verstärker"

(73) Patentinhaber : HONEYWELL MEDICAL ELECTRO-
NICS B.V.
I.B.C.-Weg 1
NL-5683 PK Best (NL)

(72) Erfinder : Peyer, Christoph
Riedlistrasse
CH-3123 Belp (CH)

(74) Vertreter : Rentzsch, Heinz et al
Honeywell Europe S.A. Holding KG Kaiserleistrasse
55 Postfach 184
D-6050 Offenbach am Main (DE)

**Beschreibung**

Die Erfindung bezieht sich auf eine EKG-Verstärkerschaltung mit Gleichstromunterdrückung durch ein RC-Hochpassglied zwischen Vorverstärker und Endverstärker. Derartige EKG-Verstärker sind beispielsweise bekannt aus « Neues aus der Technik », Nr. 1/2, 1. März 1969, S. 3. Bei Verwendung eines einzigen RC-Gliedes dieser Art entspricht die Ausgangspannung des EKG-Verstärkers als Antwort auf eine Sprungfunktion am Verstärkereingang einer Exponentialfunktion. Einerseits soll die Zeitkonstante des RC-Gliedes möglichst groß gewählt sein, damit das EKG-Signal unverzerrt dargestellt wird. Bei zu kleiner Zeitkonstante werden die flachen Wellen des EKG (P-, T- und S-Welle) abgeflacht. Andererseits führt aber eine große Zeitkonstante dazu, daß langsame Schwankungen der Elektrodenkontakt-Potentiale (Offset-Spannung) nicht mehr ausgeglichen werden, und sich in der Aufzeichnung des EKG als störende Basislinienschwankung darstellen. Bei einer großen Zeitkonstanten dauert es außerdem lange, bis die Basislinie des EKG wieder in die Nullage zurückkehrt, wenn infolge einer Störung, z. B. infolge Bewegung des Patienten oder Defibrillation eine Potentialänderung aufgetreten ist. Um beiden Anforderungen, d. h. dem Wunsch nach Verzerrungsfreiheit des dargestellten EKG-Signals und geringer Basislinienschwankung gerecht zu werden ist zu berücksichtigten, daß das eigentliche EKG-Signal stets nach relativ kurzer Zeit wieder zur Nullinie zurückkehrt. Die P- und T-Wellendauer beträgt maximal 300 ms.

Aufgabe der Erfindung ist es, eine EKG-Verstärkerschaltung zu finden, welche diesen sich teilweise widersprechenden Anforderungen besser gerecht wird, als die mit einem RC-Hochpassglied erzielte Sprungantwort in Form einer Exponentialfunktion.

Diese Aufgabe wird gelöst durch die im Anspruch 1 gekennzeichnete Erfindung. Die Übertragungsfunktion einer solchen Verstärkerschaltung hat die Eigenschaft, daß als Sprungantwort das Ausgangssignal dem Eingangssignal zunächst während 0,3 s nahezu unverändert folgt, anschließend jedoch auch bei einem länger anstehenden Eingangssignal rasch zur Nullinie zurückkehrt. Anstelle des bekannten RC-Hochpassgliedes wird hier ein RC-Filter zweiter Ordnung eingesetzt, wobei zu bemerken ist, daß auch Filter höherer Ordnung verwendet werden können, deren Impulsantwort die zuvor erwähnten Eigenschaften noch ausgeprägter aufweist. Vorteilhafte Ausgestaltungen der Erfindung ergeben sich aus den Unteransprüchen.

Die Erfindung wird nachfolgend anhand eines in den Zeichnungen wiedergegebenen Ausführungsbeispieles erläutert. Dabei zeigt

Figur 1   schematisch das Blockschaltbild einer herkömmlichen EKG-Verstärkerschaltung ;

Figur 2a   eine Sprungfunktion als Eingangsspannung $u_i$ in Abhängigkeit von der Zeit t ;

Figur 2b   die entsprechende Ausgangsspannung $u_o$ am Ausgang der Schaltungsanordnung gemäß Figur 1 ;

Figur 2c   den gewünschten Spannungsverlauf u in Abhängigkeit von der Zeit ;

Figur 3   Ausführungsbeispiel einer Schaltungsanordnung zur Erzielung eines solchen Verlaufs der Ausgangsspannung u beim Anlegen einer Sprungfunktion $u_i$ an deren Eingang.

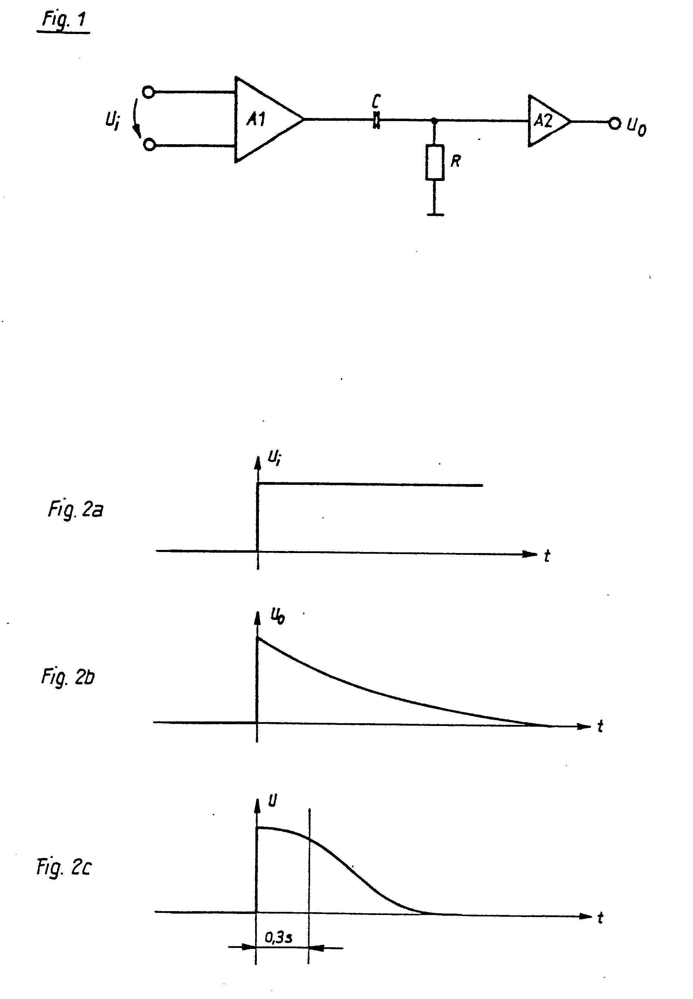

Die bekannte Schaltungsanordnung nach Figur 1 besteht aus einem Differential-Vorverstärker $A_1$, einem RC-Hochpassglied mit Kondensator C und Widerstand R, sowie einem Gleichstromverstärker $A_2$. Legt man an die Eingangsklemmen des Vorverstärkers $A_1$ eine Spannung vom Verlauf gemäß Figur 2a, so entsteht am Ausgang des Gleichstromverstärkers $A_2$ infolge der differenzierenden Wirkung des RC-Gliedes zunächst ein Spannungssprung, an den sich im Zuge der Entladung des Kondensators C über den Widerstand R ein exponentiell abklingender Spannungsverlauf gemäß Figur 2b anschließt. Ein solcher Spannungsverlauf hat bei EKG-Verstärkern aus den eingangs genannten Gründen gewisse Nachteile, weshalb für solche und ähnlichen Bedingungen unterworfene Verstärkerschaltungen ein Spannungsverlauf u(t) gemäß Figur 2c angestrebt wird. Er folgt dem Eingangssprung $u_i$ über etwa 0,3 s und klingt dann wesentlich schneller als die Exponentialkurve gemäß Figur 2b nach Null hin ab.

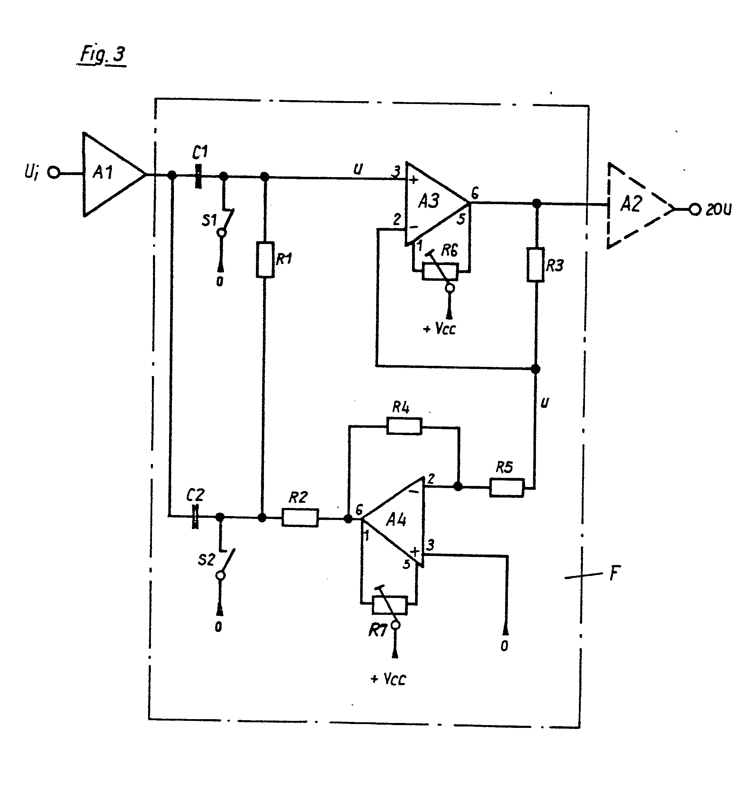

In Figur 3 ist anstelle des einfachen RC-Hochpassgliedes von Figur 1 ein RC-Filter F zweiter Ordnung zwischen die Verstärker $A_1$ und $A_2$ eingeschaltet. Dabei kann die Funktion des Gleichstromverstärkers $A_2$ durch einen im Filter F vorhandenen Operationsverstärker $A_3$ mit übernommen werden. Der Gleichstromverstärker $A_2$ ist deshalb nur gestrichelt dargestellt und kann in der Praxis entfallen.

Das Filter F enthält wiederum ein RC-Hochpassglied bestehend aus Kondensator $C_1$ und Widerstand $R_1$, welches zwischen den Vorverstärker $A_1$ und den Operationsverstärker $A_3$ eingeschaltet ist. Der Fußpunkt des Widerstands $R_1$ liegt hier nicht an Masse, sondern er ist über einen Widerstand $R_2$ an den Ausgang eines Rückführverstärkers $A_4$ angeschlossen, dessen invertierender Eingang 2 über die Reihenschaltung zweier Widerstände $R_3$ und $R_5$ an den Ausgang des Operationsverstärkers $A_3$ angeschlossen ist. Zwischen dem Verbindungspunkt der Widerstände $R_1$ und $R_2$ und dem Ausgang des Vorverstärkers $A_1$ liegt ferner ein zweiter Kondensator $C_2$. Der Widerstand $R_3$ ist zugleich als Rückführwiderstand zwischen den Ausgang und den invertierenden Eingang des Operationsverstärkers $A_3$ geschaltet, während dem Operationsverstärker $A_4$ hierzu ein besonderer Rückführwiderstand $R_4$ zugeordnet ist. Die Widerstände $R_6$ und $R_7$ dienen zum Offset-Abgleich der beiden Operationsverstärker.

Zunächst sei angenommen, daß der Operationsverstärker $A_3$ als Einheitsverstärker einen Spannungsverstärkungsfaktor « 1 » hat und eine unendlich hohe Eingangsimpedanz aufweist. Unter

2

dieser Voraussetzung entfallen die Widerstände $R_3$ und $R_5$ zwischen Ausgang des Verstärkers $A_3$ und invertierendem Eingang 2 des Operationsverstärkers $A_4$. Der Operationsverstärker $A_4$ im Rückführzweig hat einen Verstärkungsfaktor $-g$. Damit läßt sich für das aus den Widerständen $R_1$ und $R_2$, den Kondensatoren $C_1$ und $C_2$, sowie den Operationsverstärkern $A_3$ und $A_4$ bestehende Filternetzwerk die folgende Differentialgleichung für die Ausgangsspannung $u$ in Abhängigkeit von der Eingangsspannung $u_1$ aufstellen :

$$R_1 C_1 R_2 C_2\, \ddot{u} + (R_2 C_2 + R_2 C_1 + R_1 C_1)\, \dot{u} + (g + 1)\, u = (R_1 C_1 + R_2 C_1 + R_2 C_2)\, \dot{u}_1 + R_1 C_1 R_2 C_2\, \ddot{u}_1 \qquad (1)$$

Ist $u_1$ eine Sprungfunktion gemäß Fig. 1a, so gilt :

$u_1 = 0$ für $t < 0$, $u_1 = u_s$ für $t \geqslant 0$
$u_1 = \ddot{u}_1 = 0$ für $t \neq 0$

und die Differentialgleichung 1 lautet, wenn man noch $R_1 C_1 = \tau_1$, $R_2 C_2 = \tau_2$ und $R_2 C_1 = \tau_{12}$ setzt :

$$\ddot{u} + \frac{\tau_1 + \tau_{12} + \tau_2}{\tau_1 \cdot \tau_2}\, \dot{u} + \frac{g + 1}{\tau_1 \cdot \tau_2} \cdot u = 0 \text{ für } t > 0$$

Mit der Substitution $\hfill (2)$

$$\frac{\tau_1 + \tau_{12} + \tau_2}{\tau_1 \cdot \tau_2} = 2a \qquad \text{und} \qquad \frac{g + 1}{\tau_1 \cdot \tau_2} = w_o^2$$

schreibt sich die Differentialgleichung wie folgt :

$$\ddot{u} + 2a \cdot \dot{u} + w_o^2 \cdot u = 0 \qquad (3)$$

Dies ist die Differentialgleichung einer gedämpften Schwingung.

Damit das Ausgangssignal nach einem Eingangsspannungssprung möglichst rasch wieder zur Nullinie zurückkehrt, muss die Schwingung aperiodisch gedämpft sein. Die Bedingung für aperiodische Dämpfung lautet :

$$a^2 - w_o^2 = 0$$

wählt man als Spezialfall : $R_1 = R_2$ und $C_1 = C_2$, so wird mit $\tau_1 = \tau_2 = \tau_{12} = \tau$ :

$$2a = \frac{3}{\tau} \qquad \text{und} \qquad w_o^2 = \frac{g + 1}{\tau^2}$$

$$a^2 - w_o^2 = \frac{9}{4\,\tau^2} - \frac{g + 1}{\tau^2} = 0$$

und somit $g = 1{,}25$

Die Lösung der Differentialgleichung für diesen aperiodischen Fall lautet :

$$u = K_1 \cdot e^{-\frac{3}{2\tau} \cdot t} + K_2 \cdot t \cdot e^{-\frac{3}{2\tau} \cdot t}. \qquad (4)$$

Die Integrationszeitkonstanten $K_1$ und $K_2$ bestimmen sich aus den Anfangsbedingungen. Unmittelbar nach dem Spannungssprung $(t = 0)$ gilt : $u = u_s$ und $\dot{u} = 0$. Damit wird

$$K_1 = u_s \qquad \text{und} \qquad K_2 = \frac{3}{2\,\tau} \cdot u_s$$

und die Gleichung (4) lautet : $\hfill (5)$

$$u = u_s \left( e^{-\frac{3}{2\tau} \cdot t} + \frac{3}{2\,\tau} \cdot t \cdot e^{-\frac{3}{2\tau} \cdot t} \right).$$

3

Wie bereits erwähnt, übernimmt der Operationsverstärker $A_3$ zugleich die Aufgabe des Gleichstromverstärkers $A_2$ der bekannten Schaltungsanordnung gemäß Fig. 1 und hat deshalb nicht eine Verstärkungsgrad « 1 » sondern beispielsweise einen Verstärkungsfaktor 20. Folglich wird die Rückführspannung von seinem Ausgang 6 dem Rückführverstärker $A_4$ nicht unmittelbar sondern über die Widerstände $R_3$ und $R_5$ zugeführt.

Die Schaltungsanordnung gemäß Figur 3 zeigt ferner zwei Kontakte $S_1$ und $S_2$, bei deren Schließen die Kondensatoren $C_1$ und $C_2$ schnell entladen werden. Damit wird die Ausgangsspannung augenblicklich auf die Nullinie zurückgeführt. Die beiden Schalter können von Hand mit Hilfe einer Taste betätigt werden, oder auch automatisch beim Umschalten der EKG-Ableitungen oder wenn die Ausgangsspannung einen gewissen Schwellwert überschreitet.

In der Praxis kann es vorteilhaft sein, wenn die Schwingung etwas weniger als aperiodisch gedämpft ist. Die Sprungantwort hat dann stets nach einer bestimmten Zeit eine Nullstelle. Der Zeitpunkt des Nulldurchgangs ist unabhängig von der Amplitude des Sprunges. Man erreicht damit, daß das Signal auch nach einer starken Störung rasch wieder in den Nullinienbereich zurückkehrt und damit auf einem Bildschirm oder einem EKG-Schreiber wieder sichtbar wird. Dies ist dann von Bedeutung, wenn ein Patient defibrilliert wurde und anschließend das EKG weiterhin sichtbar aufgezeichnet werden soll. Voraussetzung ist, daß ein Überschwingen so klein gehalten wird, daß das Anzeigesignal den Aufzeichnungsbereich nicht sofort wieder verläßt.

Die Lösung der Differentialgleichung (3) für den Fall einer gedämpften harmonischen Schwingung lautet :

$$u = e^{-at} [K_1 \cdot \cos (wt) + K_2 \sin (wt)] \qquad (6)$$

mit der Kreisfrequenz $w = \sqrt{w_0^2 - a^2}$.

Mit der Anfangsbedingung $\dot{u} = 0$ und $u = u_s$ für $t = 0$, bestimmen sich die Integrationskonstanten zu

$$K_1 = u_s, \quad K_2 = a/w \cdot u_s$$

womit sich Gleichung (6) wie folgt schreibt :

$$u = u_s \cdot e^{-at} [\cos (wt) + a/w \cdot \sin (wt)] \qquad (7)$$

Setzt man hier z. B.
$R_1 C_1 = R_2 C_2 = \tau = 1,25\,s$ und $g = 2$, so folgt $a = 3/2\,\tau = 1,2\,s^{-1}$
$w_0^2 = g + 1/\tau^2 = 1,92\,s^{-2}$
$w = \sqrt{w_0^2 - a^2} = 0,693$
$u = u_s\, e^{-1,2t} [\cos 0,693\,t + 1,732 \sin 0,693\,t]$

Diese Funktion hat ihre erste Nullstelle bei $t = 3,78$ Sekunden. Das maximale Überschwingen tritt bei $t = 4,53\,s$ auf und nimmt den Wert $u = -0,004\,4\,u_s$ an.

**Patentansprüche**

1. EKG-Verstärkerschaltung mit Gleichstromunterdrückung durch ein erstes RC-Hochpassglied zwischen Vorverstärker und Folgeverstärker, gekennzeichnet durch folgende Merkmale :
a) der Reihenschaltung von Kondensator ($C_1$) und Widerstand ($R_1$) des ersten RC-Hochpassgliedes ($R_1$, $C_1$) ist ein zweiter Kondensator ($C_2$) parallel geschaltet ;
b) die Reihenschaltung eines invertierenden Rückführverstärkers ($A_4$) und eines Widerstandes ($R_2$) ist zwischen den Ausgang des Folgeverstärkers ($A_3$) und den Verbindungspunkt von zweitem Kondensator ($C_2$) und Widerstand ($R_1$) des ersten RC-Gliedes ($R_1$, $C_1$) eingeschaltet.

2. Schaltungsanordnung nach Anspruch 1, dadurch gekennzeichnet, daß Folge- und Rückführverstärker ($A_3$, $A_4$) als Operationsverstärker ausgebildet sind.

3. Schaltungsanordnung nach Anspruch 2, dadurch gekennzeichnet, daß der Folgeverstärker ($A_3$) zugleich als Endverstärker ($A_2$) wirksam und sein Ausgang (6) über einen Spannungsteiler ($R_3$, $R_5$) an den invertierenden Eingang (2) des Rückführverstärkers ($A_4$) angeschlossen ist.

4. Schaltungsanordnung nach einem der Ansprüche 1 bis 3, gekennzeichnet durch eine solche Dimensionierung der Komponenten der die beiden Kondensatoren ($C_1$, $C_2$), die beiden Widerstände ($R_1$, $R_2$) sowie den Folgeverstärker ($A_3$) und den Rückführverstärker ($A_4$) umfassenden Filterschaltung (F), daß das Ausgangssignal (u) als Antwort auf ein Eingangs-Sprungsignal ($u_i$) eine aperiodisch gedämpfte Schwingung ist.

5. Schaltungsanordnung nach einem der Ansprüche 1 bis 3, gekennzeichnet durch eine solche Dimensionierung der Komponenten der die beiden Kondensatoren ($C_1$, $C_2$), die beiden Widerstände ($R_1$, $R_2$) sowie den Folgeverstärker ($A_3$) und den Rückführverstärker ($A_4$) umfassenden Filterschaltung (F), daß das Ausgangssignal (u) als Antwort auf ein Eingangs-Sprungsignal ($u_i$) eine unteraperiodisch gedämpfte Schwingung ist.

6. Schaltungsanordnung nach Anspruch 5, gekennzeichnet durch je einen Endladeschalter ($S_1$, $S_2$) für jeden der beiden Kondensatoren ($C_1$, $C_2$).

**Claims**

1. E.C.G. amplifier circuit with direct current suppression by means of a first RC high pass filter network between a preamplifier and a subsequent amplifier, characterized by the following features :
a) A second capacitor ($C_2$) is connected in parallel to the series circuit consisting of the capacitor ($C_1$) and the resistor ($R_1$) of the first RC high pass filter network ($R_1$, $C_1$) ;
b) the series circuit consisting of an inverting feedback amplifier ($A_4$) and a resistor ($R_2$) is connected between the output of the subsequent amplifier ($A_3$) and the junction between the second capacitor ($C_2$) and the resistor ($R_1$) of the first RC network ($R_1$, $C_2$).

2. Circuit according to claim 1, characterized in that the subsequent amplifier and the feedback amplifier ($A_3$, $A_4$) are operational amplifiers.

3. Circuit according to claim 2, characterized in that the subsequent amplifier ($A_3$) simultaneously operates as power amplifier ($A_2$) and its output (6) is connected *via* a voltage dividing circuit ($R_3$, $R_5$) to the inverting input (2) of the feedback amplifier ($A_4$).

4. Circuit according to one of the claims 1 to 3, characterized by such a selection of the components of the filter circuit (F) comprising the two capacitors ($C_1$, $C_2$), the two resistors ($R_1$, $R_2$), the subsequent amplifier ($A_3$) and the feedback amplifier ($A_4$), that the output signal (u) in response to an input step signal ($u_i$) is an aperiodically attenuated oscillation.

5. Circuit according to one of the claims 1 to 3, characterized by such a selection of the components of the filter circuit (F) comprising the two capacitors ($C_1$, $C_2$), the two resistors ($R_1$, $R_2$), the subsequent amplifier ($A_3$) and the feedback amplifier ($A_4$), that the output signal (u) in response to an input step signal ($u_i$) is a subperiodically attenuated oscillation.

6. Circuit according to claim 5, characterized by a discharge switch ($S_1$, $S_2$) for each of the two capacitors ($C_1$, $C_2$).

**Revendications**

1. Circuit amplificateur pour un appareil électrocardiographe comportant un système de suppression à courant continu constitué par un filtre RC passe-haut monté entre un pré-amplificateur et un amplificateur asservi, caractérisé en ce qu'il comporte les caractéristiques suivantes :
a) le filtre RC passe-haut ($R_1$, $C_1$), constitué par un condensateur ($C_1$) monté en série avec une résistance ($R_1$), comporte en outre un second condensateur ($C_2$) associé en parallèle au condensateur et à la résistance précités ;
b) un amplificateur de rétro-action ($A_4$) et une résistance ($R_2$) associés en série sont montés entre la sortie de l'amplificateur asservi ($A_3$) et le point de raccordement du second condensateur ($C_2$) avec la résistance ($R_1$) du premier filtre RC ($R_1$, $C_1$).

2. Circuit selon la revendication 1, caractérisé en ce que l'amplificateur asservi et l'amplificateur à rétro-action ($A_3$, $A_4$) sont constitués par des amplificateurs opérationnels.

3. Circuit selon la revendication 2, caractérisé en ce que l'amplificateur asservi ($A_3$) joue également le rôle d'amplificateur de sortie ($A_2$), sa sortie (6) étant raccordée par l'intermédiaire d'un diviseur de tension ($R_3$, $R_5$) à l'entrée d'inversion (2) de l'amplificateur à rétro-action ($A_4$).

4. Circuit selon l'une des revendications 1 à 3, caractérisé en ce qu'on choisit les valeurs des composants constituant les deux condensateurs ($C_1$, $C_2$), les deux résistances ($R_1$, $R_2$), ainsi que l'amplificateur asservi ($A_3$) et l'amplificateur à rétro-action ($A_4$), du circuit de filtrage (F), de manière à obtenir, sous l'effet d'un signal d'entrée constitué par une tension à augmentation brusque ($u_i$), un signal de sortie (u) constitué par une oscillation à amortissement apériodique.

5. Circuit selon l'une des revendications 1 à 3, caractérisé en ce qu'on choisit les valeurs des composants constituant les deux condensateurs ($C_1$, $C_2$), les deux résistances ($R_1$, $R_2$), ainsi que l'amplificateur asservi ($A_3$) et l'amplificateur à rétro-action ($A_4$), du circuit de filtrage (F), de manière à obtenir, sous l'effet d'un signal d'entrée constitué par une tension à augmentation brusque ($u_i$), un signal de sortie (u) constitué par une oscillation à amortissement sous-apériodique.

6. Circuit selon la revendication 5, caractérisé en ce qu'il comporte deux contacteurs de décharge ($S_1$, $S_2$), associés chacun à l'un des deux condensateurs ($C_1$, $C_2$).

_Fig. 1_

$U_i$  A1  C  R  A2  $U_0$

Fig. 2a

$U_i$

t

Fig. 2b

$U_0$

t

Fig. 2c

$U$

0,3s

t

1

Fig. 3